# EUROPÄISCHE PATENTSCHRIFT

(11) **EP 0 773 221 B1**
(45) Veröffentlichungstag und Bekanntmachung des Hinweises auf die Patenterteilung: **05.04.2000**
(21) Anmeldenummer: 96116403.5
(22) Anmeldetag: 14.10.1996
(51) Int. Cl.: C07D 475/04, A61K 31/495

(54) **Stabile kristalline Tetrahydrofolsäure-Salze**
Stable crystalline tetrahydrofolic acid salts
Sels cristallins stables de l'acide tétrahydrofolique

(30) Priorität: 07.11.1995 CH 314595
(43) Veröffentlichungstag der Anmeldung: 14.05.1997
(73) Patentinhaber: EPROVA Aktiengesellschaft, CH-8200 Schaffhausen (CH)
(72) Erfinder: Müller, Hans Rudolf, 8207 Schaffhausen (CH); Moser, Rudolf, 8200 Schaffhausen (CH); Ulmann, Martin, 8447 Dachsen (CH); Ammann, Thomas, 8460 Marthalen (CH)

(56) Entgegenhaltungen:
- EP-A- 0 348 641
- EP-A- 0 495 204
- EP-A- 0 535 710
- EP-A- 0 600 460
- EP-A- 0 682 026
- US-A- 5 124 452

## Beschreibung

Die Erfindung betrifft kristalline N-[4-[[(2-amino-1,4,5,6,7,8-hexahydro-4-oxo-(6S)-, -(6R)- und -(6R,S)-pteridinyl)methyl]amino]benzoyl]-L-glutaminsäure-Salze (im folgenden Tetrahydrofolsäure-Salze genannt), deren Verwendung sowie ein Verfahren zu deren Herstellung.

Tetrahydrofolsäure-Derivate enthalten 2 asymmetrische Zentren. Dabei liegt aufgrund der Synthese dieser Derivate aus Folsäure, der N-(Pteroyl)-L-glutaminsäure, das im Glutaminsäure-Rest enthaltene optisch aktive C-Atom in der L-Form vor, während das üblicherweise durch Hydrierung der Doppelbindung in 5,6-Stellung des Pteroyl-Restes entstandene optisch aktive C-Atom in Position 6 in der racemischen, der (6R,S)-Form, vorliegt. Synthetische Derivate der Tetrahydrofolsäure bestehen demnach aus einer 1:1 Mischung von 2 Diastereomeren.

Als Arzneimittel werden Tetrahydrofolate vorwiegend als Calcium-Salz der 5-Formyl-5,6,7,8-tetrahydrofolsäure (Leucovorin) oder der 5-Methyl-5,6,7,8-tetrahydrofolsäure verwendet zur Behandlung von megaloblastischer Folsäure-Anämie, als Antidot zur Verstärkung der Verträglichkeit von Folsäure-Antagonisten, speziell von Aminopterin und Methotrexat in der Krebstherapie ("Antifolate rescue"), zur Verstärkung des therapeutischen Effektes von fluorierten Pyrimidinen und der Behandlung von Autoimmunkrankheiten wie Psoriasis und rheumatischer Arthritis, zur Verstärkung der Verträglichkeit von bestimmten Antiparasitika, etwa Trimethoprim-Sulfamethoxazol sowie zur Verminderung der Toxizität von Dideazatetrahydrofolaten in der Chemotherapie. Im Organismus können die einzelnen Tetrahydrofolsäure-Derivate ineinander umgewandelt werden (Folate-Cycle). Tetrahydrofolsäure nimmt dabei eine zentrale Rolle ein. Ebenfalls dient Tetrahydrofolsäure als Grundsubstanz zur Herstellung diverser Tetrahydrofolsäure-Derivate.

Es bestehen verschiedene Verfahren zur Herstellung von Tetrahydrofolsäure und deren Derivaten. Speziell zu erwähnen sind EP 0 600 460, EP 0 348 641, US 5 124 452, EP 0 535 710, EP 0 682 026 und EP 0 495 204.

Die direkte Verwendung von Tetrahydrofolsäure-Salzen als Arzneimittel sowie als Grundsubstanz zur Herstellung diverser Tetrahydrofolsäure-Derivate scheiterte bisher an der Schwierigkeit, Tetrahydrofolsäure-Salze mit einer für einen pharmazeutischen Wirkstoff akzeptablen Reinheit herzustellen und an der extremen Instabilität der Tetrahydrofolsäure, wobei insbesondere die hohe Oxidationsempfindlichkeit auffällt [siehe dazu auch A.L. Fitzhugh, Pteridines 4(4), 187-191 (1993)]. Dabei ist anzumerken, dass bei der im pharmazeutischen Bereich am häufigsten benutzten parenteralen Verwendung von Tetrahydrofolaten ein zumindest annähernd neutraler pH-Wert der verabreichten Lösung zwingende Voraussetzung ist. Salze der Tetrahydrofolate entsprechen bei dieser Verwendung deshalb der bevorzugten Anwendungsform. Zur Überwindung der Instabilität der Tetrahydrofolsäure wurden verschiedene Methoden wie ein möglichst vollständiger Sauerstoffausschluss oder die Zugabe von Oxidationsschutzmitteln wie Ascorbinsäure angewendet. Ein vollständiger Sauerstoffausschluss ist jedoch bei der pharmazeutischen Verwendung kaum, und wenn dann nur mit sehr grossem Aufwand zu realisieren, und die Zugabe von Oxidationsschutzmitteln ist ebenfalls bei der pharmazeutischen Anwendung nicht immer möglich. Bisher konnte demnach noch kein technisch gangbares Verfahren gefunden werden, das für die Herstellung von Tetrahydrofolsäure-Salzen mit einer hohen Reinheit und einer ausreichenden Stabilität geeignet ist und somit die pharmazeutische Verwendung von Tetrahydrofolsäure-Salzen ermöglichen würde.

Es wurde nun überraschend gefunden, dass sich (6S)-, (6R)- oder (6R,S)-Tetrahydrofolsäure-Salze mit einer hohen Reinheit und einer ausgezeichneten Stabilität erhalten lassen, indem das entsprechende Salz der optisch reinen (6S)- oder optisch reinen (6R)-, angereicherten (6S)- oder angereicherten (6R)-, oder auch (6R,S)- Tetrahydrofolsäure kristallisiert wird. Die so erhaltenen kristallinen (6S)-, (6R)- und/oder (6R,S)-Tetrahydrofolsäure-Salze sind in guter Form bei Raumtemperatur praktisch unbeschränkt stabil. Sie sind geeignet als Bestandteil oder als Ausgangsmaterial zur Herstellung von Arzneimittelformen, bzw. als Ausgangsmaterial zur technischen Herstellung anderer Tetrahydrofolsäure-Derivate mit einer hohen Reinheit.
Gegenstand der Erfindung sind demnach kristalline Salze der (6R,S)-, (6S)- und (6R)-Tetrahydrofolsäure. Als Salze der Tetrahydrofolsäure zur Kristallisation bevorzugt eingesetzt werden Erdalkalisalze, im speziellen das Magnesium- oder Calcium-Salz.

Ein weiterer Gegenstand der Erfindung ist das Verfahren zur Herstellung kristalliner Salze der (6R,S)-, (6S)- und (6R)-Tetrahydrofolsäure, welches dadurch gekennzeichnet ist, dass man das entsprechende Salz der Tetrahydrofolsäure kristallisiert. Die Kristallisation der Tetrahydrofolsäure-Salze erfolgt dabei in Wasser oder einem Gemisch aus Wasser und einem mit Wasser mischbaren organischen Lösungsmittel wie wasserlöslichen Alkoholen, z. B. Methanol, Ethanol, n-Propanol, iso-Propanol, Ethylenglycol, einer niedrigen aliphatischen wasserlöslichen Carbonsäure, z. B. Ameisensäure, Essigsäure, Milchsäure oder wasserlöslichen Amiden, z. B. Formamid, Dimethylformamid, Dimethylacetamid, 1-Methylpyrrolidon, 2-Methylpyrrolidon, 2-Piperidinon, bei einem pH-Wert zwischen 7 und 10. Es gibt keine besonderen Einschränkungen bezüglich der Art des eingesetzten Lösungsmittels und des Mischungsverhältnisses, da kristalline Tetrahydrofolsäure-Salze generell tiefere Löslichkeiten als die entsprechenden amorphen Formen aufweisen.

Die Kristallisation wird bevorzugt bei erhöhter Temperatur, im speziellen zwischen 50°C und 90°C oder aus verdünnten Lösungen, im speziellen zwischen 1% und 10% durchgeführt.
Die Kristallisation der (6S)-, (6R)- und (6R,S)-Tetrahydrofolsäure-Salze erfolgt spontan oder durch Animpfen mit entsprechendem kristallinem Tetrahydrofolsäure-Salz.

Als Ausgangsmaterial der Kristallisation eignet sich bevorzugt amorphe oder kristalline, reine (6S)- oder (6R)-Tetrahydrofolsäure, es kann jedoch auch racemische (6R,S)-Tetrahydrofolsäure wie auch angereicherte (6S)- oder (6R)-Tetrahydrofolsäure eingesetzt werden. Es eignen sich dabei sowohl isolierte Feststoffe wie z. B. (6R,S)-Tetrahydrofolsäure, (6S)-Tetrahydrofolsäure-Schwefelsäure und Sulfonsäure-Additionssalze hergestellt nach EP-495'204, wie auch in situ durch katalytische Hydrierung oder durch Borhydrid-Reduktion aus Folsäure hergestellte Tetrahydrofolsäure als Ausgangsmaterial.

Durch den Einsatz von amorpher oder teilkristalliner optisch reiner Tetrahydrofolsäure oder deren Salzen als Ausgangsmaterial für die Kristallisation erhält man durch das beschriebene Verfahren kristalline Tetrahydrofolsäure-Salze von bisher nie erreichter Reinheit (> 98%) und einer bisher ebenfalls nie erreichten Stabilität.

Die Erfindung betrifft auch die Verwendung kristalliner (6S)-, (6R)- und/oder (6R,S)-Tetrahydrofolsäure-Salze als Bestandteil zur Herstellung von Arzneimitteln oder zur Herstellung anderer Tetrahydrofolsäure-Derivate, da kristalline (6S)-, (6R)- und (6R,S)-Tetrahydrofolsäure-Salze aufgrund ihrer ausgezeichneten Stabilität in fester Form eine zeitlich praktisch unbeschränkt gleichbleibende sehr gute Qualität behält. Ebenso betrifft die Erfindung auch pharmazeutische Zubereitungen enthaltend kristalline (6S)-, (6R)- und/oder (6R,S)-Tetrahydrofolsäure-Salze. Die Herstellung der pharmazeutischen Zubereitung erfolgt nach bekannten Verfahren wie z. B. Lyophilisierung. Die Löslichkeit der kristallinen Tetrahydrofolsäure-Salze in Wasser bei 20°C liegt unter 1 mg/ml Die Anwendung erfolgt analog zur Anwendung von bekannten Substanzen aus dem Gebiet der Tetrahydrofolate wie z. B. 5-Formyl-5,6,7,8-tetrahydrofolsäure.

Im weiteren betrifft die Erfindung ein Verfahren zur Trennung von (6R,S)-Tetrahydrofolsäure-Magnesium-Salz in die beiden Diastereomeren (6S)- und (6R)-Tetrahydrofolsäure-Magnesium-Salze durch fraktionierte Kristallisation. Dieses Verfahren ist sehr einfach und ergiebig. Man erhält bereits bei der ersten Kristallisation eines rohen racemischen (6R,S)-Tetrahydrofolsäure-Magnesium-Salzes kristallines (6R)-Tetrahydrofolsäure-Magnesium-Salz mit einem (6R)-Anteil von über 95% in Enantiomerenausbeuten von über 50%. Durch weitere Kristallisationen unter analogen Bedingungen können kristalline (6S)- und (6R)-Tetrahydrofolsäure-Magnesium-Salze mit einer höheren Isomerenreinheit erhalten werden.

### Beispiele zur Illustrierung der Erfindung

Die in den Beispielen angegebenen Gehalte an Tetrahydrofolsäure-Salz und die Isomerenanteile wurden jeweils mit HPLC bestimmt.

### Beispiel 1 (Stabilitäten)

Zur Stabilitätsbestimmung der kristallinen (6S)- und (6R)-Tetrahydrofolsäure-Salze wurden die Substanzen zusammen mit Vergleichsmustern bei 25°C und 60% Feuchte unter Stickstoff, bzw. bei 4°C unter Luft gelagert. In periodischen Abständen wurde der verbleibende Gehalt an Tetrahydrofolsäure-Salz gemessen und im Vergleich zum Ausgangswert angegeben.

| | | **Belastungszeit in Monaten** | | | | | |
|---|---|---|---|---|---|---|---|
| | | **0** | **1/2** | **1** | **2** | **6** | **9** |
| kristallines Ca-Salz der (6S)-Tetrahydrofolsäure | 25°C, 60%, N₂ | 100% | 98.5% | 97.2% | 96.8% | 97.7% | 97.7% |
| | 4°C, Luft | 100% | 95.5% | 92.4% | 89.2% | 82.3% | 76.0% |
| kristallines Ca-Salz der (6R)-Tetrahydrofolsäure | 25°C, 60%, N₂ | 100% | 97.8% | 96.1% | 98.0% | 97.1% | 96.8% |
| | 4°C, Luft | 100% | 85.9% | 79.0% | 73.0% | 69.3% | 69.2% |
| kristallines Ca-Salz der (6R,S)-Tetrahydrofolsäure | 25°C, 60%, N₂ | 100% | 99.3% | 98.0% | 99.0% | 97.8% | 98.7% |
| | 4°C, Luft | 100% | 96.5% | 94.2% | 89.0% | 86.6% | 86.2% |

Kristalline Tetrahydrofolsäure-Salze sind auch nach längerer Belastungszeit noch sehr hell. Im Gegensatz dazu verfärben sich amorphe Proben rasch sehr stark. Amorphes Calcium-Salz der (6R,S)-Tetrahydrofolsäure weist nach einer Belastungszeit von einem Monat bei 4°C unter Luft einen im Vergleich zu kristallinem Calcium-Salz der (6R,S)-Tetrahydrofolsäure um 8% tieferen Gehalt auf.

### Beispiel 2 (Pulverröntgendiagramme)

Zur Charakterisierung der strukturellen Eigenschaften (Kristallinität) der kristallinen Tetrahydrofolsäure-Salze wurden von diesen Substanzen Pulverröntgendiagramme (Beugungsspektren) aufgenommen.

Kristalline (6S)-, (6R)- und (6R,S)-Tetrahydrofolsäure-Salze ergeben gut aufgelöste Spektren mit scharfen Banden und niedrigem Untergrund. Die Spektren weisen auf hohe kristalline Anteile hin.

### Beispiel 3

8.2 g (6R,S)-Tetrahydrofolsäure werden unter Stickstoff in 100 ml Wasser enthaltend 1 g Thioglycerin aufgeschlämmt, mit Natronlauge 30% auf pH 3.3 gestellt und mit einer Lösung von 3.8 g Calciumchlorid in 4 g Wasser versetzt. Die entstandene Lösung weist einen pH-Wert von 9.3 auf. Nach 20 Stunden Rühren bei Raumtemperatur wird die entstandene Suspension, die einen pH-Wert von 10.0 aufweist, genutscht und der Rückstand mit wenig Wasser überwaschen.

Nach dem Trocknen erhält man 7.7 g leicht rötliches, kristallines Calcium-Salz der (6R,S)-Tetrahydrofolsäure mit einem (6S)-Anteil von 51.1% und einem Gehalt von 96% Fläche.

Die Löslichkeit des erhaltenen Produktes liegt bei weniger als 1 mg/ml Wasser (25°C).

### Beispiel 4

28.6 g (6R,S)-Tetrahydrofolsäure werden unter Stickstoff in 114 ml Wasser aufgeschlämmt, mit Natronlauge 30% auf pH 7.5 gestellt und mit einer Lösung von 48.5 g Calciumchlorid in 500 ml Wasser versetzt. Die entstandene gummiartige Masse wird auf 90°C erwärmt. Nach ca 1 Stunde Rühren erhält man eine leuchtend gelbe Suspension, die heiss genutscht und mit wenig Wasser überwaschen wird.

Nach dem Trocknen erhält man 17.3 g beiges, kristallines Calcium-Salz der (6R,S)-Tetrahydrofolsäure mit einem (6S)-Anteil von 50.9% und einem Gehalt von 94% Fläche.

### Beispiel 5

4.0 g (6R,S)-Tetrahydrofolsäure werden unter Stickstoff in 40 ml Wasser enthaltend 0.4 g Thioglycerin aufgeschlämmt, mit Natronlauge 30% auf pH 8.5 gestellt und bei 50°C mit 2.0 g Calciumacetat versetzt. Das aus der entstandenen Lösung langsam auskristallisierende, beige Produkt wird abgenutscht und mit Wasser überwaschen.

Nach dem Trocknen erhält man 3.64 g kristallines Calcium-Salz der (6R,S)-Tetrahydrofolsäure mit einem (6S)-Anteil von 50.5% und einem Gehalt von 94.6% (als Salz, auf trockene Substanz bezogen). Der Calcium-Anteil beträgt 1.12 Äquivalent.

### Beispiel 6

12.0 g (6S)-Tetrahydrofolsäure werden unter Stickstoff in 60 ml Wasser enthaltend 0.6 g Thioglycerin aufgeschlämmt, mit Natronlauge 50% auf pH 7.5 gestellt und bei 85°C mit einer Lösung von 22.5 g Calciumchlorid in 20 ml Wasser versetzt. Nach 2 Stunden rühren bei 85°C wird das auskristallisierte Produkt abgenutscht und mit Wasser überwaschen.

Nach dem Trocknen erhält man 12.9 g kristallines Calcium-Salz der (6S)-Tetrahydrofolsäure mit einem (6S)-Anteil von 99.9% und einem Gehalt von 96.8% Fläche. Die Löslichkeit des so erhaltenen Produktes in Wasser bei 50°C und bei einem pH-Wert von 6 beträgt 0.12%.

### Beispiel 7

Durch den Einsatz von 12.0 g (6R)-Tetrahydrofolsäure und analoger Behandlung wie im Beispiel 6 beschrieben erhält man 13.8g kristallines Calcium-Salz der (6R)-Tetrahydrofolsäure mit einem (6R)-Anteil von 99.0% und einem Gehalt von 93% Fläche. Die Löslichkeit des so erhaltenen Produktes in Wasser bei 50°C und bei einem pH-Wert von 6 beträgt 0.07%.

### Beispiel 8

40.0 g (6S)-Tetrahydrofolsäure werden unter Stickstoff in 160 ml Wasser aufgeschlämmt und bei 0-5°C mit Ammoniak 25% auf pH 9.8 gestellt. Zur entstandenen Lösung werden 34 g Magnesiumchlorid in 34 ml Wasser zugegeben. Nach dem Einhalten des pH-Wertes auf 7.0 und der Zugabe von 200 ml Ethanol wird das auskristallisierte, beige Produkt abgenutscht und mit Ethanol/Wasser überwaschen.

Nach dem Trocknen erhält man 37.0 g kristallines Magnesium-Salz der (6S)-Tetrahydrofolsäure mit einem (6S)-Anteil von 99.4% und einem Gehalt von 91.7% Fläche.

### Beispiel 9

40.0 g (6R)-Tetrahydrofolsäure werden unter Stickstoff in 400 ml Wasser enthaltend 4 g Thioglycerin aufgeschlämmt und mit 6.0 g Magnesiumhydroxid und 60.0 g Magnesiumacetat versetzt. Mit Ammoniak 25% wird der pH-Wert bei 50°C auf 9.0 eingestellt. Nach dem Abkühlen auf 20°C erhält man eine gelartige Masse, die beim Erwärmen auf 35°C in eine dünne Suspension übergeht. Die Suspension wird bei 35°C genutscht und mit Wasser überwaschen.

Nach dem Trocknen erhält man 18.0g kristallines Magnesium-Salz der (6R)-Tetrahydrofolsäure mit einem (6R)-Anteil von 99.4% und einem Gehalt von 92.0% Fläche.

### Beispiel 10

20.0 g (6R,S)-Tetrahydrofolsäure werden unter Stickstoff in 200 ml Wasser enthaltend 2 g Thioglycerin aufgeschlämmt, mit 2.7 g Magnesiumhydroxid versetzt und auf 50°C aufgeheizt. Nach der Zugabe von 30 g Magnesiumacetat wird der pH-Wert mit Ammoniak 25% auf 7.3 eingestellt, die Lösung auf 20°C gekühlt und über Nacht gerührt. Die erhaltene Suspension wird abgenutscht und mit Wasser überwaschen.

Nach dem Trocknen erhält man 5.0 g kristallines Magnesium-Salz der (6R)-Tetrahydrofolsäure mit einem (6R)-Anteil von 94.8% und einem Gehalt von 97.1% Fläche.

### Beispiel 11

28.0 g (6R,S)-Tetrahydrofolsäure werden in 110 ml Wasser und 75ml Methanol enthaltend 18 g Thioglycerin aufgeschlämmt, mit 9.5 g Magnesiumhydroxid versetzt und bei 50°C mit Ammoniak 25% auf einen pH-Wert von 9.3 eingestellt. Die nach dem Kühlen auf -5°C entstandene feine Suspension wird abgenutscht und mit kaltem Methanol/Wasser-Gemisch überwaschen.

Nach dem Trocknen erhält man 10.5g kristallines Magnesium-Salz der (6R,S)-Tetrahydrofolsäure mit einem (6S)-Anteil von 49.5% und einem Gehalt von 95.9% Fläche.

### Beispiel 12

4.0 g (6R,S)-Tetrahydrofolsäure werden unter Stickstoff in 16 ml Wasser aufgeschlämmt, mit Ammoniak 25% auf einen pH-Wert von 9.7 eingestellt und mit einer Lösung von 3.2 g Magnesiumchlorid in 3.2 ml Wasser versetzt. Die entstandene klare Lösung wird in 200 ml Ethanol eingetragen. Die erhaltene hellgelbe Suspension wird nach 2 Stunden rühren bei 5°C abgenutscht und mit kaltem Ethanol/Wasser-Gemisch überwaschen.

Nach dem Trocknen erhält man 4.5 kristallines Magnesium-Salz der (6R,S)-Tetrahydrofolsäure mit einem (6S)-Anteil von 49.3% und einem Gehalt von 90.3% Fläche.

## Patentansprüche

1. Kristallines Calcium-Salz der (6R,S)- und (6S)-Tetrahydrofolsäure.

2. Kristallines Calcium-Salz der (6R)-Tetrahydrofolsäure, erhältlich durch ein Verfahren, das dadurch gekennzeichnet ist, dass man (6R)-Tetrahydrofolsäure in Wasser oder einem Gemisch aus Wasser und einem mit Wasser mischbaren organischen Lösungsmittel bei einem pH-Wert zwischen 7 und 10 kristallisiert.

3. Kristallines Magnesium-Salz der (6R,S)-, (6S)- und (6R)-Tetrahydrofolsäure.

4. Verfahren zur Herstellung von kristallinem Calcium- oder Magnesium-Salz der (6R,S)- (6S)- und/oder (6R)-Tetrahydrofolsäure, dadurch gekennzeichnet, dass man (6R,S)-, (6S)- oder (6R)-Tetrahydrofolsäure in Wasser oder einem Gemisch aus Wasser und einem mit Wasser mischbaren organischen Lösungsmittel bei einem pH-Wert zwischen 7 und 10 kristallisiert.

5. Verfahren nach Anspruch 4, dadurch gekennzeichnet, dass die Kristallisation bei erhöhter Temperatur und/oder aus verdünnten Lösungen durchgeführt wird.

6. Verfahren nach Anspruch 4, dadurch gekennzeichnet, dass als mit Wasser mischbares organisches Lösungsmittel Essigsäure oder ein wasserlöslicher Alkohol eingesetzt wird.

7. Verwendung von kristallinem Calcium- oder Magnesium-Salz der (6R,S)-, (6S)- oder (6R)-Tetrahydrofolsäure als Bestandteil zur Herstellung von Arzneimitteln.

8. Pharmazeutische Zubereitungen enthaltend kristallines Calcium- oder Magnesiumsalz der (6R,S)-, (6S)- oder (6R)-Tetrahydrofolsäure.

## Claims

1. A crystalline calcium salt of (6R,S)- and (6S)- tetrahydrofolic acid.

2. A crystalline calcium salt of (6R)-tetrahydrofolic acid, obtainable by a method which is characterised in that (6R)-tetrahydrofolic acid is crystallised in water or in a mixture of water and a water-miscible organic solvent at a pH between 7 and 10.

3. A crystalline magnesium salt of (6R,S)-, (6S)- and (6R)- tetrahydrofolic acid.

4. A method of preparing a crystalline calcium or magnesium salt of (6R,S)-, (6S)- and/or (6R)-tetrahydrofolic acid, characterised in that (6R,S)-, (6R)- or (6S)-tetrahydrofolic acid is crystallised in water or in a mixture of water and a water-miscible organic solvent at a pH between 7 and 10.

5. A method according to claim 4, characterised in that crystallisation is effected at elevated temperature and/or from dilute solutions.

6. A method according to claim 4, characterised in that acetic acid or a watersoluble alcohol is used as the solvent miscible with water.

7. The use of a crystalline calcium or magnesium salt of (6R,S)-, (6S)- or (6R)-tetrahydrofolic acid as an ingredient for the production of drugs.

8. Pharmaceutical preparations containing a crystalline calcium or magnesium salt of (6R,S)-, (6S)- or (6R)-tetrahydrofolic acid.

## Revendications

1. Sel de calcium cristallin de l'acide (6R,S)- et (6S)-tétrahydrofolique.

2. Sel de calcium cristallin de l'acide (6R)-tétrahydrofolique, pouvant être obtenu par un procédé caractérisé en ce qu'on cristallise de l'acide (6R)-tétrahydrofolique dans de l'eau ou dans un mélange d'eau et d'un solvant organique miscible à l'eau, à un pH compris entre 7 et 10.

3. Sel de magnésium cristallin de l'acide (6R,S)-, (6S)- et (6R)-tétrahydrofolique.

4. Procédé de préparation du sel de calcium ou de magnésium cristallin de l'acide (6R,S)-, (6S)- et/ou (6R)-tétrahydrofolique, caractérisé en ce qu'on cristallise de l'acide (6R,S)-, (6S)- ou (6R)-tétrahydrofolique dans de l'eau ou dans un mélange d'eau et d'un solvant organique miscible à l'eau, à un pH compris entre 7 et 10.

5. Procédé selon la revendication 4, caractérisé en ce que la cristallisation est mise en oeuvre à haute température et/ou dans des solutions diluées.

6. Procédé selon la revendication 4, caractérisé en ce qu'on utilise en tant que solvant organique miscible à l'eau de l'acide acétique ou un alcool soluble dans l'eau.

7. Utilisation de sel de calcium ou de magnésium cristallin de l'acide (6R,S)-, (6S)- ou (6R)-tétrahydrofolique en tant que constituant pour la fabrication de médicaments.

8. Préparations pharmaceutiques contenant un sel de calcium ou de magnésium cristallin de l'acide (6R,S)-, (6S)- ou (6R)-tétrahydrofolique.
